# EUROPEAN PATENT SPECIFICATION

(11) **EP 2 652 174 B1**
(45) Date of publication and mention of the grant of the patent: **18.03.2015**
(21) Application number: 11797002.0
(22) Date of filing: 14.12.2011
(51) Int. Cl.: C25B 3/08

(54) **PROCESS FOR THE ELECTROCHEMICAL FLUORINATION OF ORGANIC COMPOUNDS**
VERFAHREN ZUR ELEKTROCHEMISCHEN FLUORIERUNG ORGANISCHER VERBINDUNGEN
PROCÉDÉ DE FLUORATION ÉLECTROCHIMIQUE DE COMPOSÉS ORGANIQUES

(30) Priority: 15.12.2010 EP 10195196
(43) Date of publication of application: 23.10.2013
(73) Proprietor: BASF SE, 67056 Ludwigshafen (DE)
(72) Inventor: AUST, Nicola Christiane, 68199 Mannheim (DE); MALKOWSKY, Itamar Michael, 67346 Speyer (DE)
(86) International application number: PCT/EP2011/072682
(87) International publication number: WO 2012/080292

(56) References cited:
- WO-A1-98/00580
- US-A- 4 146 443
- MASARU HASEGAWA: "Regioselective anodic monofluorination of ethers,lactones,carbonates,and esters using ionic liquid fluoride salts.", JOURNAL OF THE ELECTROCHEMICAL SOCIETY, vol. 153, no. 10, 2 August 2006 (2006-08-02), pages D162-D166, XP002670543,

## Description

### FIELD OF THE INVENTION

The present invention relates to a process for the electrochemical fluorination of an organic compound, wherein a liquid reaction medium comprising the organic compound and a fluorinating agent is being electrolyzed.

### BACKGROUND OF THE INVENTION

Fluorochemicals (in particular partly fluorinated and perfluorinated organic compounds) are commercially valuable and useful chemical materials. Fluorochemicals can exhibit various useful properties, e.g., they may be inert, nonpolar, hydrophobic, oleophobic, etc. As such, fluorochemicals have found a wide variety of applications, e.g. as oil, water and stain resistant chemicals, as refrigerants and heat exchange agents or as solvents and cleaning agents. Fluorinated heterocyclic compounds have found wide use as pharmaceuticals and agrochemicals due to their high and specific physiological activity. Because of the versatility of fluorochemicals and consequently a strong demand for these materials, there is a continuing need for new and/or improved methods for their preparation.

### DESCRIPTION OF THE RELATED ART

One well-known industrial process for preparing fluorochemical compounds is the electrochemical perfluorination process developed in the 1940s by John Simons (J. H. Simons, J. Electrochem. Soc. 1949, 95, 47 - 52), often referred to as Simons fluorination or electrochemical fluorination (ECF). According to this method, an electrolyte solution containing a mixture of liquid anhydrous hydrogen fluoride and an organic compound intended to be fluorinated is subjected to electrolysis. Generally, the Simons process is practiced at nickel electrodes with a constant current passed through the electrolyte. The current passing through the electrolyte causes one or more of the hydrogen atoms of the organic compound to be replaced by fluorine atoms.

A further principal electrochemical method for introducing fluorine directly into organic compounds is the electrolysis in salt melts, e.g. in potassium fluoride/hydrogen fluoride melts, using porous carbon anodes. This method is also known as Phillips process.

For partial fluorine substitution it is known to employ electrolysis in fluoride ion solutions, namely HF complexes using platinum electrodes. Though other electrode materials are described in the patent and non-patent literature, platinum is the electrode of choice in most actual partial electrochemical fluorinations (E. Hollitzer, P. Sartori, Chem.-Ing.-Tech. 1986, 58, 31 - 38).

One advantage of platinum electrodes is their high degree of stability against HF complexes. J. H. H. Meurs and W. Eilenberg describe in Tetrahedron, Vol. 47, No. 4/5, pp. 705 - 714, 1991, methods for the oxidative fluorination in amine-HF mixtures. Electrode materials which were found to be stable using (C₂H₅)₃N• 3 HF in the electrofluorination of benzene were vitreous carbon (which rapidly degrades in electrolytes with higher HF content) and platinum deposited on a copper base. The lifetime of graphite electrodes did not exceed 10 minutes.

Although fluorination processes with platinum electrodes allow reactions with certain selectivity, it is generally difficult to introduce one or more fluorine atoms only or predominantly at the desired position in an organic compound by electrolysis. In many cases the obtained selectivity and/or conversion rate is low. To overcome these problems different fluorinating agents have been developed, wherein HF is complexed with amine bases, like (C₂H₅)₃N• m HF, wherein m denotes the moles of HF molecules the complex comprises (e.g. 2, 3, 4, 5 or 6). The HF complexes can be employed as electrolyte and fluorinating agent and at the same time also as solvent. Thus, the electrochemical fluorination with HF complexes can be performed in the absence of solvents. In a different variant, the electrochemical fluorination with HF complexes is performed in an aprotic solvent.

The complexation of HF leads often to an increased selectivity. On the other hand, the reactivity of the fluorinating agent and the conversion rate of the fluorination reaction are in many case decreased by the complexation of HF. In some cases low selectivity and low conversion rates are improved by the use of HF complexes with a high HF content, e.g. (C₂H₅)₄NF• 5 HF. Nevertheless, the success of this strategy depends on the substrate employed for the electrochemical fluorination. Further, an important drawback of HF complexes with a high HF content is their higher corrosivity compared to complexes with a lower HF content, like (C₂H₅)₃N• 3 HF. There is a need for materials and in particular electrodes that have an enhanced lifetime if they are employed in this kind of electrochemical fluorination.

It is known that the choice of an appropriate solvent, e.g. dimethoxyethane or other ether-containing additives with coordination ability to cations and anodic stability, can improve the selectivity of the ECF (S. Inagi, T. Sawamura, T. Fuchigami, Electrochemistry Communications 10 (2008), 1158 - 1160). Also the use of additives like ionic liquids can have a positive effect on the selectivity and/or conversion rate of the ECF. T. Fuchigami describes in Journal of Fluorine Chemistry 2007, 128, 311 - 316 solvent effects on the selectivity of the electrochemical fluorination of organic compounds.

Drawbacks in the industrial utilization are that these additives cause additional costs and have to be separated after the electrolysis.

There is still a great need for an effective method that allows the electrochemical fluorination of organic compounds with a high selectivity and/or a high conversion rate of the fluorination reaction. Preferably, this method should also allow a long lifetime of the employed electrodes.

It has now been found that, surprisingly gas diffusion layers (GDL) are advantageously suitable for the use as the anode in electrochemical fluorination processes. They show a high degree of stability against most fluorinating agents and in particular amine-HF complexes. Electrochemical fluorination processes wherein a GDL electrode is employed are usually characterized by a high selectivity in view of a certain product. Further, electrochemical fluorination processes wherein a GDL electrode is employed usually show high conversion rates. Advantageously, the process of the invention allows the use of HF complexes with a lower HF content, like (C₂H₅)₃N• 3 HF. Even with HF complexes with a lower HF content and without addition of solvents or other additives conversion and/or selectivity of the electrochemical fluorination are improved.

The closest prior art is represented by US 4146443.

### SUMMARY OF THE INVENTION

The invention is defined in claims 1 and 7.

In a first aspect, the invention provides a process for the electrochemical fluorination of an organic compound comprising the steps of:
a) providing an organic compound comprising at least one hydrogen atom bound to a carbon atom;
b) providing, in an electrochemical cell comprising a cathode and an anode, a liquid reaction medium comprising the organic compound and a fluorinating agent;
c) electrolyzing the liquid reaction medium to cause replacement of at least a part of said hydrogen atoms with fluorine atoms,
characterized in that a gas diffusion layer is used as the anode.

In a further aspect, the invention provides the use of a gas diffusion layer electrode in a process for the electrochemical fluorination of an organic compound to improve the selectivity and/or conversion rate of the fluorination reaction.

### DETAILED DESCRIPTION

In the context of the present invention, the expression C₁-C₆-alkyl comprises straight-chain or branched C₁-C₆-alkyl groups. Examples of C₁-C₆-alkyl groups are methyl, ethyl, n-propyl, isopropyl, n-butyl, isobutyl, sec-butyl, tert-butyl, n-pentyl, neo-pentyl and n-hexyl.

The above remarks regarding alkyl also apply to the alkyl moiety in alkoxy.

In the context of the present invention, the term "cycloalkyl" denotes a cycloaliphatic radical having usually from 3 to 12 carbon atoms, preferably 5 to 8 carbon atoms, such as cyclopropyl, cyclobutyl, cyclopentyl, cyclohexyl, cycloheptyl, cyclooctyl, norbornyl, bicyclo[2.2.2]octyl or adamantyl.

In the context of the present invention, the term C₆-C₁₀-aryl refers to mono- or polycyclic aromatic hydrocarbon radicals. C₆-C₁₀-aryl is preferably phenyl or naphthyl.

It is a critical feature of the process according to the invention to employ an electrochemical cell (electrolysis cell) that comprises at least one GDL electrode as anode.

GDLs are known to a person skilled in the art and are commercially available. Suitable GDLs are described inter alia in US 4,748,095, US 4,931,168 and US 5,618,392. The teaching of those documents is incorporated herein by reference. Suitable commercially available GDLs are e.g. of the H2315 series from Freudenberg FCCT KG, Höhner Weg 2 - 4, 69465 Weinheim, Germany.

A GDL generally comprises a fibre layer or substrate and a microporous layer (MPL) consisting of carbon particles attached to each other. The degree of hydrophobization can vary in such a way that wetting and gas permeability can be adjusted.

GDL electrodes for the process of the invention preferably do not contain a catalyst supported on the surface of the electrode.

The use of at least one GDL electrode in the process of the invention has a positive effect on at least one of the following parameters: selectivity of the electrofluorination, conversion rate of the fluorination reaction, space-time yield, and service life of the cell. While not being bound to any theory it is assumed, that intermediates of the fluorination reaction of the organic compound, e.g. cation-radicals generated during the anodic oxidation step, are stabilized by the GDL electrode.

As cathode any electrode suitable for electrochemical fluorination processes can be used. A person skilled in the art can determine which electrode is suitable. Preferably, the cathode is selected from Pt, Pb, Ni, steel and GDL electrodes.

### Step a)

The organic compound provided in step a) is a compound that comprises at least one hydrogen atom directly bound to a carbon atom that can be substituted by fluorine under the conditions of the electrochemical fluorination. It is of course also possible to employ a mixture of organic compounds. Suitable are organic compounds that in combination with at least one fluorinating agent and optionally solvents and/or additives allow the formation of a liquid reaction medium with ionic conductivity so that electrolysis can be applied to cause fluorination of the organic compound(s).

The liquid reaction medium within the electrochemical fluorination cell includes an electrolyte phase comprising HF and an amount of organic compound solubilized therein. The organic compound can be in the form of a liquid, solid, or gas, and can be introduced to the hydrogen fluoride as appropriate for its physical state.

The organic compound can comprise functional groups that are essentially stable under the reaction conditions. Suitable functional groups comprise carbonyl, thiocarbonyl, ester, thioester, amide, oxycarbonyloxy, urethane, urea, hydroxyl, sulfonyl, sulfinate, sulfonate, sulfate, ether, amine, nitrile, etc. and combinations thereof.

The organic compound provided in step a) is selected from compounds of the general formula I 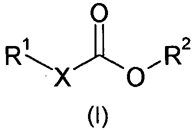 wherein
- X: is O, N-R³ or CR⁴R⁵,
- R¹: is selected from C₁-C₆-alkyl, C₁-C₆-alkoxy-C₁-C₆-alkyl, formyl, C₁-C₆-alkylcarbonyl and C₁-C₆-alkyloxycarbonyl,
wherein R¹ may also be C₁-C₆-alkoxy if X is a CR⁴R⁵ group,
wherein R¹ may also be C₁-C₆-alkylcarbonyloxy if X is a N-R³ or CR⁴R⁵ group,
- R²: is selected from hydrogen, C₁-C₆-alkyl, C₁-C₆-alkoxy-C₁-C₆-alkyl, C₆-C₁₀-aryl-C₁-C₆-alkyl, C₃-C₁₂-cycloalkyl and C₆-C₁₀-aryl,
- R³: is selected from hydrogen, C₁-C₆-alkyl, C₁-C₆-alkoxy-C₁-C₆-alkyl, formyl, C₁-C₆-alkylcarbonyl and C₁-C₆-alkyloxycarbonyl,
- R⁴ and R⁵: are independently selected from hydrogen, C₁-C₆-alkyl, C₁-C₆-alkoxy-C₁-C₆-alkyl and C₁-C₆-alkoxy,
- or R¹ and R²: together with the X-(C=O)-O group to which they are bound form a 5-to 7-membered heterocyclic ring, which may contain at least one additional heteroatom or heteroatom containing group, selected from O, S, NR^{a} or C=O, wherein R^{a} is selected from hydrogen, alkyl, cycloalkyl and aryl,
- or X: is a CR⁴R⁵ group and R¹ and R⁴ together with the carbon atom to which they are bound form a 3 to 7 membered carbocyclic ring.

Preferably, X is O, CH₂ or NR³, wherein R³ is C₁-C₄-alkyl or C₁-C₄-alkylcarbonyl. In particular, X is NR³, wherein R³ is methyl, ethyl, n-propyl, n-butyl, methylcarbonyl or ethylcarbonyl.

In a preferred embodiment, R¹ and R² together with the X-(C=O)-O group to which they are bound form a 5 to 7 membered heterocyclic ring, which may contain at least one additional heteroatom or heteroatom containing group, selected from O, S, NR^{a} or C=O, wherein R^{a} is selected from hydrogen, alkyl, cycloalkyl or aryl. In this embodiment, X is preferably O, CH₂ or N-R³, wherein R³ is C₁-C₄-alkyl or C₁-C₄-alkylcarbonyl. Further, in this embodiment R¹ and R² together are selected from groups of the formulae -CH₂-CH₂-, -CH₂-CH₂-CH₂- and -CH(CₓH₂ₓ₊₁)-CH₂-, wherein x is 1, 2, 3 or 4.

In a further preferred embodiment, R¹ is selected from C₁-C₄-alkyl. In particular R¹ is selected from methyl, ethyl, n-propyl and n-butyl.

In a further preferred embodiment, X is a CH₂ group and R¹ is selected from hydrogen, C₁-C₄-alkyl, C₁-C₄-alkoxy, formyl, C₁-C₄-alkylcarbonyl and C₁-C₄-alkyloxycarbonyl. In particular, X is a CH₂ group and R¹ is selected from hydrogen, methyl, ethyl, n-propyl, n-butyl, methoxy, ethoxy, n-propyloxy, n-butyloxy, formyl, methylcarbonyl, ethylcarbonyl, methoxycarbonyl and ethoxycarbonyl.

In a further preferred embodiment, X is a CR⁴R⁵ group and R¹ and R⁴ together with the carbon atom to which they are bound form a 3 to 7 membered carbocyclic ring. In this embodiment, R¹ and R⁴ together with the carbon atom to which they are bound form a cyclopropyl ring. Further, in this embodiment, preferably R⁵ is hydrogen.

Preferably, R² is selected from C₁-C₄-alkyl. In particular R² is selected from methyl, ethyl, n-propyl and n-butyl.

Examples of suitable organic compounds are:

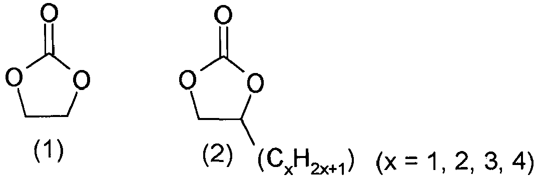

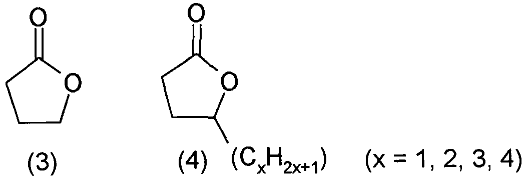

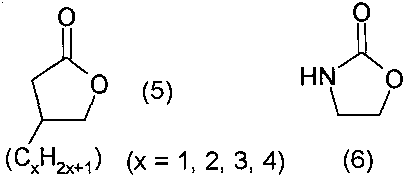

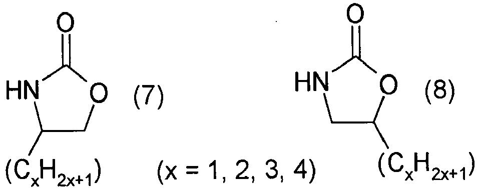

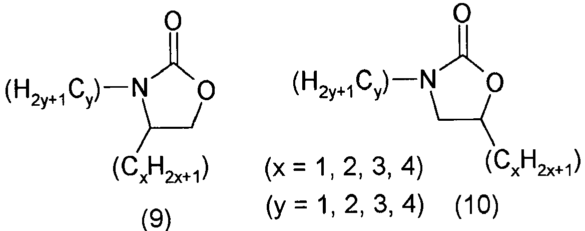

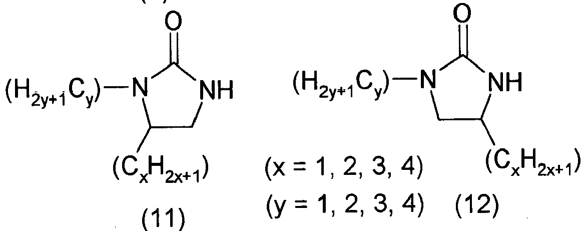

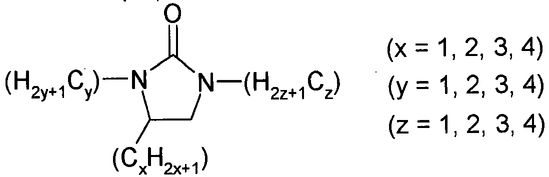

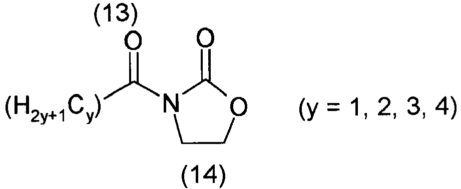

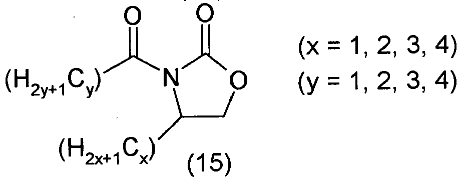

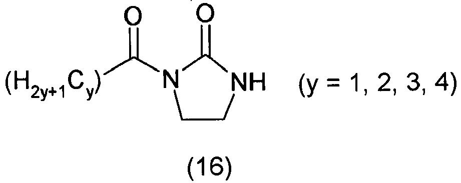

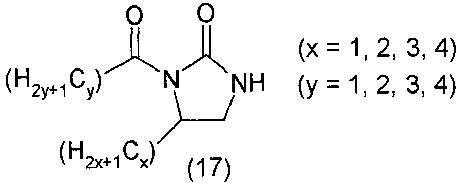

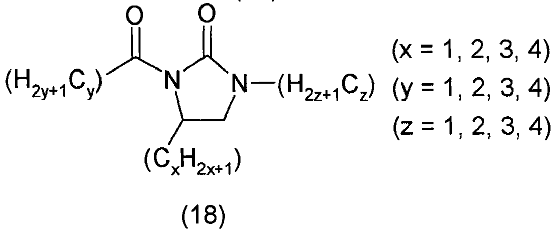

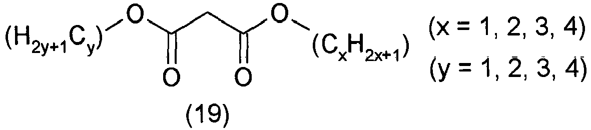

The organic compound provided in step a) is preferably ethylene carbonate or propylene carbonate (4-methyl-1,3-dioxolan-2-one).

### Step b)

In step b) of the process according to the invention, a liquid reaction medium comprising the organic compound and a fluorinating agent is provided.

The fluorinating agent employed in step b) can be any agent or mixtures of agents which is capable to substitute a hydrogen atom with a fluorine atom during the electrochemical fluorination.

Preferably, the fluorinating agent is selected from complexes of HF with amine bases. Preferred are complexes of HF with trialkylamines, ammonium fluorides, pyridine and mixtures thereof. In particular, the HF-complex is selected from polyhydrofluoride complexes of trialkylamines and tetraalkylammoniumfluorides.

Advantageously, the process of the invention allows the use of HF complexes with a low HF content. Thus, in a preferred embodiment, (C₂H₅)₃N• 3 HF is employed as the H F-complex.

The molar ratio of fluorinating agent (with regard HF) to organic compound is preferably in the range from 1:1 to 99:1 (fluorinating agent: organic compound), more preferably from 50:25 to 99:1.

In the context of the present invention, the expression "liquid reaction medium" denotes a reaction medium that comprises a liquid phase under the reaction conditions of the fluorination reaction. This liquid phase contains a sufficient amount of the organic compound to allow electrochemical fluorination. It is not necessary that the liquid phase contains the organic compound in form of a homogeneous solution, as long as a sufficient amount of the organic compound is brought in contact with the electrodes of the electrochemical cell, in particular the anode. Thus, the liquid reaction medium may contain the organic compound in form of a homogeneous solution, colloidal solution, molecularly disperse solution, emulsified phase or disperse phase. Finally, it is also possible to introduce a gaseous stream containing the organic compound into the liquid reaction medium.

In comparison to processes known from the prior art, the process according to the invention does not require any additional solvents or additives to establish a fluorination reaction with high conversion rates and good selectivity. In particular, the HF complex employed as fluorinating agent may also function as conducting salt (electrolyte) and/or as solvent.

Preferably, the liquid reaction medium provided in step b) comprises in essence no additional solvents and other additives, i.e. the proportion of solvents and other additives is below 1 % by weight, based on the total weight of the liquid reaction medium.

If the reaction medium provided in step b) contains an organic solvent, it is preferably selected from acetonitrile, ethers, halogenated alkanes, sulfolane, ionic liquids and mixtures thereof.

If at least one HF complex is employed as fluorinating agent, the liquid reaction medium is generally sufficiently electrically conductive to allow electrolysis in an amount sufficient to result in fluorination of the organic compound.

An overview on the construction possibilities of electrolysis cells that are suitable as electrochemical fluorination cells for the process of the invention can be found, for example, in D. Pletcher, F. Walsh, Industrial Electrochemistry, 2nd Edition, 1990, London, pp. 60ff.

Suitable electrochemical cells for the electrochemical fluorination are undivided cells and divided cells. An undivided cell usually comprises only one electrolyte portion; a divided cell has two or more such portion. The individual electrodes can be connected in parallel (monopolar) or serially (bipolar). In a suitable embodiment, the electrochemical cell employed for the fluorination is a monopolar cell comprising a GDL anode and a cathode. In a further suitable embodiment, the electrochemical cell employed for the fluorination is a cell having bipolar connection of the stacked electrodes.

In a preferred embodiment, the electrochemical fluorination cell is a plate-and-frame cell. Plate-and-frame cells employed in the process of the invention comprise at least one GDL electrode. This type of cell is composed essentially of usually rectangular electrode plates and frames which surround them. They can be made of polymer material, for example polyethylene, polypropylene, polyvinyl chloride, polyvinylidene fluoride, PTFE, etc. The electrode plate and the associated frame are frequently joined to each other to form an assembly unit. By pressing a plurality of such plate-and-frame units together, a stack which is assembled according to the constructional fashion of filter presses is obtained. Yet further frame units, for example for receiving spacing gauzes, etc. can be inserted in the stack.

### Step c)

The process according to the invention can be performed according to known methods for the electrochemical fluorination of organic compounds by electrolyzing the liquid reaction medium in order to cause replacement of at least a part of the carbon bound hydrogen atoms with fluorine atoms, with the proviso that the employed electrochemical fluorination cell comprises at least a GDL anode.

One or more anodes and one or more cathodes are placed in the liquid reaction medium. According to the invention, at least the anode is a GDL electrode. An electric potential (voltage) is established between the anode(s) and cathode(s), resulting in an oxidation reaction (primarily fluorination, i.e., replacement of one or more carbon bound hydrogen atoms with carbon bound fluorine atoms) at the anode, and a reduction reaction (primarily hydrogen evolution) at the cathode.

Preferably, the electrochemical fluorination is performed with a constant current applied; i.e. at a constant voltage and a constant current flow. It is of course also possible, to interrupt the electric current through a current cycle, as described in US 6,267,865.

Preferably, the current density employed in step c) is in a range of from 10 to 250 mA/cm².

The fluorination products can be separated from the reaction medium by customary methods, preferably by distillation. The distillation of the reaction discharge can be carried out by customary methods known to those skilled in the art. Suitable apparatuses for the fractionation by distillation comprise distillation columns such as tray columns, which can be provided with bubble caps, sieve plates, sieve trays, packings, internals, valves, side offtakes, etc. Dividing wall columns, which may be provided with side offtakes, recirculations, etc., are especially suitable. A combination of two or more than two distillation columns can be used for the distillation. Further suitable apparatuses are evaporators such as thin film evaporators, falling film evaporators, Sambay evaporators, etc, and combinations thereof.

The following examples are intended for further illustration of the present invention.

### Example 1: 4-fluoroethylene carbonate

In a 100 ml undivided electrolysis cell 30,2 g ethylene carbonate and 40 g triethylamine tris(hydrogenfluoride) were electrolyzed for 2F using a GDL electrode (H2315 IX11 CX108 from Freudenberg, 10 cm²) as anode and a Pb cathode (10 cm²). The current density was 100 mA/cm². Afterwards the electrolysis output was stirred with 60 g ice water, three times extracted with 50 ml ethyl acetate and dried over magnesium sulphate. After removing the ethyl acetate by distillation a crude mixture showed 66% conversion of ethylene carbonate, 61% selectivity towards 4-fluoroethylene carbonate which resulted in a yield of 40%.

### Comparative example C-1:

In a 100 ml undivided electrolysis cell 30,2 g ethylene carbonate and 40 g triethylamine tris(hydrogenfluoride) were electrolyzed for 2 F using platinum electrodes (10 cm²) as anode and cathode. The current density was 100 mA/cm². Afterwards the electrolysis output was stirred with 60 g ice water, three times extracted with 50 ml ethyl acetate and dried over magnesium sulphate. After removing the ethyl acetate by distillation a crude mixture showed 28% conversion of ethylene carbonate, 24% selectivity towards 4-fluoroethylene carbonate which resulted in a yield of 7%.

### Examples 2 and 3:

According to the procedure described in example 1 the electrochemical fluorination of ethylene carbonate was repeated with different pairs of electrodes.

**Table 1 electrochemical fluorination of different organic compounds**

| Example | anode | cathode | fluorinating agent | conversion [%] | selectivity [%] | yields [%] |
|---|---|---|---|---|---|---|
| 1 | GDL | Pb | Et₃N * 3 HF | 66 | 61 | 40 |
| C-1⁺⁾ | Pt | Pt | Et₃N * 3 HF | 28 | 24 | 7 |
| 2 | GDL | GDL | Et₃N * 3 HF | 55 | 54 | 30 |
| 3 | GDL | Pt | Et₃N * 3 HF | 67 | 60 | 40 |

| | | | | | | |
|---|---|---|---|---|---|---|
| ⁺⁾ comparative | | | | | | |

### Example 4: 4-fluoro-4-methyl-1,3-dioxolan-2-one (4-fluoropropylene carbonate)

4-fluoro-4-methyl-1,3-dioxolan-2-one was synthesized by electrochemical fluorination of 4-methyl-1,3-dioxolan-2-one using essentially the process of example 1. After removing the ethyl acetate by distillation the obtained reaction product showed 58% conversion of propylene carbonate. The GC analysis shows 23 area % 4-fluoro-4-methyl-1,3-dioxolan-2-one. Due to the missing GC factor for the product the selectivity towards 4-fluoropropylene carbonate can only be estimated to be higher than 40%.

## Claims

1. A process for the electrochemical fluorination of an organic compound comprising the steps of:
a) providing an organic compound comprising at least one hydrogen atom bound to a carbon atom which is selected from compounds of the general formula I 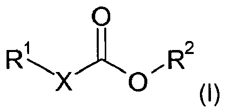 wherein
X is O, N-R³ or CR⁴R₅,
R¹ is selected from C₁-C₆-alkyl, C₁-C₆-alkoxy-C₁-C₆-alkyl, formyl, C₁-C₆-alkylcarbonyl and C₁-C₆-alkyloxycarbonyl,
wherein R¹ may also be C₁-C₆-alkoxy if X is a CR⁴R⁵ group,
wherein R¹ may also be C₁-C₆-alkylcarbonyloxy if X is a N-R³ or CR⁴R⁵ group,
R² is selected from hydrogen, C₁-C₆-alkyl, C₁-C₆-alkoxy-C₁-C₆-alkyl, C₆-C₁₀-aryl-C₁-C₆-alkyl, C₃-C₁₂-cycloalkyl and C₆-C₁₀-aryl,
R³ is selected from hydrogen, C₁-C₆-alkyl, C₁-C₆-alkoxy-C₁-C₆-alkyl, formyl, C₁-C₆-alkylcarbonyl and C₁-C₆-alkyloxycarbonyl,
R⁴ and R⁵ are independently selected from hydrogen, C₁-C₆-alkyl, C₁-C₆-alkoxy-C₁-C₆-alkyl and C₁-C₆-alkoxy,
or R¹ and R² together with the X-(C=O)-O group to which they are bound form a 5- to 7-membered heterocyclic ring, which may contain at least one additional heteroatom or heteroatom containing group, selected from O, S, NR^{a} or C=O, wherein R^{a} is selected from hydrogen, alkyl, cycloalkyl and aryl,
or X is a CR⁴R⁵ group and R¹ and R⁴ together with the carbon atom to which they are bound form a 3 to 7 membered carbocyclic ring;
b) providing, in an electrochemical cell comprising a cathode and an anode, a liquid reaction medium comprising the organic compound and a fluorinating agent which is a HF-complex selected from polyhydrofluoride complexes of trialkylamines and tetraalkylammoniumfluorides;
c) electrolyzing the liquid reaction medium to cause replacement of at least a part of said hydrogen atoms with fluorine atoms,
**characterized in that** a gas diffusion layer electrode is used as the anode, wherein the gas diffusion layer electrode comprises a fibre layer or substrate and a microporous layer (MPL) consisting of carbon particles attached to each other.

2. A process according to claim 1, wherein (C₂H₅)₃N• 3 HF is employed as the HF-complex.

3. A process according to either of claims 1 or 2, wherein the cathode is selected from gas diffusion layer (GDL), Pt, Pb, steel and Ni electrodes.

4. A process according to any of claims 1 to 3, wherein no additional solvents or additives are added to the liquid reaction medium.

5. A process according to any of the preceding claims, wherein the organic compound provided in step a) is ethylene carbonate or propylene carbonate.

6. A process according to any of the preceding claims, wherein the current density employed in step c) ranges from 10 to 250 mA/cm².

7. The use of a gas diffusion layer electrode, wherein the gas diffusion layer electrode comprises a fibre layer or substrate and a microporous layer (MPL) consisting of carbon particles attached to each other, in a process for the electrochemical fluorination of an organic compound which is selected from compounds of the general formula I 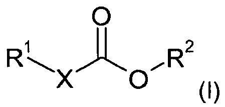 wherein
X is O, N-R³ or CR⁴R⁵,
R¹ is selected from C₁-C₆-alkyl, C₁-C₆-alkoxy-C₁-C₆-alkyl, formyl, C₁-C₆-alkylcarbonyl and C₁-C₆-alkyloxycarbonyl,
wherein R¹ may also be C₁-C₆-alkoxy if X is a CR⁴R⁵ group,
wherein R¹ may also be C₁-C₆-alkylcarbonyloxy if X is a N-R³ or CR⁴R⁵ group,
R² is selected from hydrogen, C₁-C₆-alkyl, C₁-C₆-alkoxy-C₁-C₆-alkyl, C₆-C₁₀-aryl-C₁-C₆-alkyl, C₃-C₁₂-cycloalkyl and C₆-C₁₀-aryl,
R³ is selected from hydrogen, C₁-C₆-alkyl, C₁-C₆-alkoxy-C₁-C₆-alkyl, formyl, C₁-C₆-alkylcarbonyl and C₁-C₆-alkyloxycarbonyl,
R⁴ and R⁵ are independently selected from hydrogen, C₁-C₆-alkyl, C₁-C₆-alkoxy-C₁-C₆-alkyl and C₁-C₆-alkoxy,
or R¹ and R² together with the X-(C=O)-O group to which they are bound form a 5- to 7-membered heterocyclic ring, which may contain at least one additional heteroatom or heteroatom containing group, selected from O, S, NR^{a} or C=O, wherein R^{a} is selected from hydrogen, alkyl, cycloalkyl and aryl,
or X is a CR⁴R⁵ group and R¹ and R⁴ together with the carbon atom to which they are bound form a 3 to 7 membered carbocyclic ring,
to improve the selectivity and/or conversion rate of the fluorination reaction, wherein a HF-complex selected from polyhydrofluoride complexes of trialkylamines and tetraalkylammoniumfluorides is used as the fluorinating agent and wherein the gas diffusion layer electrode is used as the anode.

8. The use according to claim 7 for the electrochemical fluorination of ethylene carbonate to obtain a fluorination product containing 4-fluoroethylene carbonate.

9. The use according to claim 7 for the electrochemical fluorination of propylene carbonate to obtain a fluorination product containing 4-fluoropropylene carbonate.

## Patentansprüche

1. Verfahren zur elektrochemischen Fluorierung einer organischen Verbindung, bei dem man:
a) eine organische Verbindung mit mindestens einem an ein Kohlenstoffatom gebundenen Wasserstoffatom bereitstellt, die aus Verbindungen der allgemeinen Formel I ausgewählt wird: 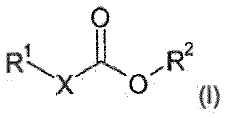 wobei
X für 0, N-R³ oder CR⁴R⁵ steht,
R¹ aus C₁-C₆-Alkyl, C₁-C₆-Alkoxy-C₁-C₆-alkyl, Formyl, C₁-C₆-Alkylcarbonyl und C₁-C₆-Alkyloxycarbonyl ausgewählt ist,
wobei R¹ auch für C₁-C₆-Alkoxy stehen kann, wenn X für eine CR⁴R⁵-Gruppe steht,
wobei R¹ auch für C₁-C₆-Alkylcarbonyloxy stehen kann, wenn X für eine N-R³- oder CR⁴R⁵-Gruppe steht,
R² aus Wasserstoff, C₁-C₆-Alkyl, C₁-C₆-Alkoxy-C₁-C₆-alkyl, C₆-C₁₀-Aryl-C₁-C₆-alkyl, C₃-C₁₂-Cycloalkyl und C₆-C₁₀-Aryl ausgewählt ist,
R³ aus Wasserstoff, C₁-C₆-Alkyl, C₁-C₆-Alkoxy-C₁-C₆-alkyl, Formyl, C₁-C₆-Alkylcarbonyl und C₁-C₆-Alkyloxycarbonyl ausgewählt ist,
R⁴ und R⁵ unabhängig aus Wasserstoff, C₁-C₆-Alkyl, C₁-C₆-Alkoxy-C₁-C₆-alkyl und C₁-C₆-Alkoxy ausgewählt sind,
oder R¹ und R² zusammen mit der X-(C=O)-O-Gruppe, an die sie gebunden sind, einen 5-bis 7-gliedrigen heterocyclischen Ring bilden, der mindestens ein zusätzliches Heteroatom bzw. mindestens eine zusätzliche heteroatomhaltige Gruppe, die aus O, S, NR^{a} oder C=O ausgewählt ist, enthalten kann, wobei R^{a} aus Wasserstoff, Alkyl, Cycloalkyl und Aryl ausgewählt ist,
oder X für eine CR⁴R⁵-Gruppe steht und R¹ und R⁴ zusammen mit dem Kohlenstoffatom, an das sie gebunden sind, einen 3- bis 7-gliedrigen carbocyclischen Ring bilden;
b) in einer elektrochemischen Zelle mit einer Kathode und einer Anode ein flüssiges Reaktionsmedium bereitstellt, das die organische Verbindung und ein Fluorierungsmittel, bei dem es sich um einen aus Polyhydrofluorid-Komplexen von Trialkylaminen und Tetraalkylammoniumfluoriden ausgewählten HF-Komplex handelt, umfasst;
c) das flüssige Reaktionsmedium elektrolysiert, wodurch mindestens ein Teil der Wasserstoffatome durch Fluoratome ersetzt wird,
**dadurch gekennzeichnet, dass** als Anode eine Gasdiffusionsschicht-Elektrode verwendet wird, wobei die Gasdiffusionsschicht-Elektrode eine Faserschicht oder ein Fasersubstrat und eine mikroporöse Schicht (MPL) aus aneinander gebundenen Kohlenstoffpartikeln umfasst.

2. Verfahren nach Anspruch 1, bei dem als HF-Komplex (C₂H₅)N·3 HF eingesetzt wird.

3. Verfahren nach Anspruch 1 oder 2, bei dem die Kathode aus Gasdiffusionsschicht(GDL)-, Pt-, Pb-, Stahl- und Ni-Elektroden ausgewählt wird.

4. Verfahren nach einem der Ansprüche 1 bis 3, bei dem keine zusätzlichen Lösungsmittel oder Additive zu den flüssigen Reaktionsmedium gegeben werden.

5. Verfahren nach einem der vorhergehenden Ansprüche, bei dem es sich bei der in Schritt a) bereitgestellten organischen Verbindung um Ethylencarbonat oder Propylencarbonat handelt.

6. Verfahren nach einem der vorhergehenden Ansprüche, bei dem die in Schritt c) eingesetzte Stromdichte im Bereich von 10 bis 250 mA/cm² liegt.

7. Verwendung einer Gasdiffusionsschicht-Elektrode, wobei die Gasdiffusionsschicht-Elektrode eine Faserschicht oder ein Fasersubstrat und eine mikroporöse Schicht (MPL) aus aneinander gebundenen Kohlenstoffpartikeln umfasst, bei einem Verfahren zur elektrochemischen Fluorierung einer organischen Verbindung, die aus Verbindungen der allgemeinen Formel I ausgewählt wird: 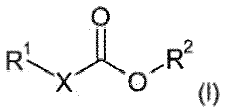 wobei
X für 0, N-R³ oder CR⁴R⁵ steht,
R¹ aus C₁-C₆-Alkyl, C₁-C₆-Alkoxy-C₁-C₆-alkyl, Formyl, C₁-C₆-Alkylcarbonyl und C₁-C₆-Alkyloxycarbonyl ausgewählt ist,
wobei R¹ auch für C₁-C₆-Alkoxy stehen kann, wenn X für eine CR⁴R⁵-Gruppe steht,
wobei R¹ auch für C₁-C₆-Alkylcarbonyloxy stehen kann, wenn X für eine N-R³- oder CR⁴R⁵-Gruppe steht,
R² aus Wasserstoff, C₁-C₆-Alkyl, C₁-C₆-Alkoxy-C₁-C₆-alkyl, C₆-C₁₀-Aryl-C₁-C₆-alkyl, C₃-C₁₂-Cycloalkyl und C₆-C₁₀-Aryl ausgewählt ist,
R³ aus Wasserstoff, C₁-C₆-Alkyl, C₁-C₆-Alkoxy-C₁-C₆-alkyl, Formyl, C₁-C₆-Alkylcarbonyl und C₁-C₆-Alkyloxycarbonyl ausgewählt ist,
R⁴ und R⁵ unabhängig aus Wasserstoff, C₁-C₆-Alkyl, C₁-C₆-Alkoxy-C₁-C₆-alkyl und C₁-C₆-Alkoxy ausgewählt sind,
oder R¹ und R² zusammen mit der X-(C=O)-O-Gruppe, an die sie gebunden sind, einen 5-bis 7-gliedrigen heterocyclischen Ring bilden, der mindestens ein zusätzliches Heteroatom bzw. mindestens eine zusätzliche heteroatomhaltige Gruppe, die aus 0, S, NR^{a} oder C=O ausgewählt ist, enthalten kann, wobei R^{a} aus Wasserstoff, Alkyl, Cycloalkyl und Aryl ausgewählt ist,
oder X für eine CR⁴R⁵-Gruppe steht und R¹ und R⁴ zusammen mit dem Kohlenstoffatom, an das sie gebunden sind, einen 3- bis 7-gliedrigen carbocyclischen Ring bilden;
zur Verbesserung der Selektivität und/oder Umsatzrate der Fluorierungsreaktion,
wobei ein aus Polyhydrofluorid-Komplexen von Trialkylaminen und Tetraalkylammoniumfluoriden ausgewählter HF-Komplex als Fluorierungsmittel verwendet wird und wobei die Gasdiffusionsschicht-Elektrode als Anode verwendet wird.

8. Verwendung nach Anspruch 7 zur elektrochemischen Fluorierung von Ethylencarbonat zum Erhalt eines 4-Fluorethylencarbonat enthaltenden Fluorierungsprodukts.

9. Verwendung nach Anspruch 7 zur elektrochemischen Fluorierung von Propylencarbonat zum Erhalt eines 4-Fluorpropylencarbonat enthaltenden Fluorierungsprodukts.

## Revendications

1. Procédé de fluoration électrochimique d'un composé organique comprenant les étapes consistant à :
a) se procurer un composé organique comprenant au moins un atome d'hydrogène lié à un atome de carbone qui est choisi parmi les composés de la formule générale I 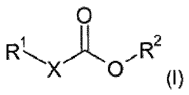 dans laquelle
X représente 0, N-R³ ou CR⁴R⁵,
R¹ est choisi parmi un alkyle en C₁-C₆, un (alcoxy en C₁-C₆)-alkyle en C₁-C₆, un formyle, un alkylcarbonyle en C₁-C₆ et un alkyloxycarbonyle en C₁-C₆,
R¹ pouvant aussi être un alcoxy en C₁-C₆ si X est un groupe CR⁴R⁵,
R¹ pouvant aussi être un alkylcarbonyloxy en C₁-C₆ si X est un groupe N-R³ ou CR⁴R⁵,
R² est choisi parmi un hydrogène, un alkyle en C₁-C₆, un (alcoxy en C₁-C₆)-alkyle en C₁-C₆, un (aryl en C₆-C₁₀)-alkyle en C₁-C₆, un cycloalkyle en C₃-C₁₂ et un aryle en C₆-C₁₀,
R³ est choisi parmi un hydrogène, un alkyle en C₁-C₆, un (alcoxy en C₁-C₆)-alkyle en C₁-C₆,un formyle, un alkylcarbonyle en C₁-C₆ et un alkyloxycarbonyle en C₁-C₆,
R⁴ et R⁵ sont indépendamment choisis parmi un hydrogène, un alkyle en C₁-C₆, un (alcoxy en C₁-C₆)-alkyle en C₁-C₆ et un alcoxy en C₁-C₆,
ou R¹et R², conjointement avec le groupe X-(C=O)-O auquel ils sont liés, forment un noyau hétérocyclique à 5 à 7 chaînons, qui peut contenir au moins un hétéroatome ou groupe contenant un hétéroatome supplémentaire, choisi parmi O, S, NR³ et C=O, R³ étant choisi parmi un hydrogène, un alkyle, un cycloalkyle et un aryle,
ou X est un groupe CR⁴R⁵ et R¹ et R⁴, conjointement avec l'atome de carbone auquel ils sont liés, forment un noyau carbocyclique à 3 à 7 chaînons ;
b) se procurer, dans une pile électrochimique comprenant une cathode et une anode, un milieu réactionnel liquide comprenant le composé organique et un agent de fluoration qui est un complexe de HF choisi parmi les complexes de polyhydrofluorure de trialkylamines et de fluorures de tétraalkylammonium ;
c) électrolyser le milieu réactionnel liquide pour provoquer le remplacement d'au moins une partie desdits atomes d'hydrogène par des atomes de fluor,
**caractérisé en ce qu'**une électrode à couche de diffusion gazeuse est utilisée comme anode,
l'électrode à couche de diffusion gazeuse comprenant une couche ou un substrat de fibres et une couche microporeuse (MPL) constituée de particules de carbone attachées les unes aux autres.

2. Procédé selon la revendication 1, dans lequel (C₂H₅)₃N•3HF est employé comme complexe de HF.

3. Procédé selon l'une ou l'autre des revendications 1 et 2, dans lequel la cathode est choisie parmi les électrodes à couche de diffusion gazeuse (GDL), de Pt, de Pb, d'acier et de Ni.

4. Procédé selon l'une quelconque des revendications 1 à 3, dans lequel aucun solvant ni additif supplémentaire n'est ajouté au milieu réactionnel liquide.

5. Procédé selon l'une quelconque des revendications précédentes, dans lequel le composé organique obtenu à l'étape a) est le carbonate d'éthylène ou le carbonate de propylène.

6. Procédé selon l'une quelconque des revendications précédentes, dans lequel la densité de courant employée à l'étape c) va de 10 à 250 mA/cm².

7. Utilisation d'une électrode à couche de diffusion gazeuse, l'électrode à couche de diffusion gazeuse comprenant une couche ou un substrat de fibres et une couche microporeuse (MPL) constituée de particules de carbone attachées les unes aux autres, dans un procédé de fluoration électrochimique d'un composé organique qui est choisi parmi les composés de la formule générale I 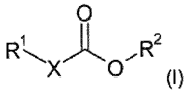 dans laquelle
X représente O, N-R³ ou CR⁴R⁵,
R¹ est choisi parmi un alkyle en C₁-C₆, un (alcoxy en C₁-C₆)-alkyle en C₁-C₆, un formyle, un alkylcarbonyle en C₁-C₆, et un alkyloxycarbonyle en C₁-C₆,
R¹ pouvant aussi être un alcoxy en C₁-C₆ si X est un groupe CR⁴R⁵,
R¹ pouvant aussi être un alkylcarbonyloxy en C₁-C₆ si X est un groupe N-R³ ou CR⁴R⁵,
R² est choisi parmi un hydrogène, un alkyle en C₁-C₆, un (alcoxy en C₁-C₆)-alkyle en C₁-C₆, un (aryl en C₆-C₁₀)-alkyle en C₁-C₆, un cycloalkyle en C₃-C₁₂ et un aryle en C₆-C₁₀,
R³ est choisi parmi un hydrogène, un alkyle en C₁-C₆, un (alcoxy en C₁-C₆)-alkyle en C₁-C₆, un formyle, un alkylcarbonyle en C₁-C₆ et un alkyloxycarbonyle en C₁-C₆,
R⁴ et R⁵ sont indépendamment choisis parmi un hydrogène, un alkyle en C₁-C₆, un (alcoxy en C₁-C₆)-alkyle en C₁-C₆ et un alcoxy en C₁-C₆,
ou R¹ et R², conjointement avec le groupe X-(C=O)-O auquel ils sont liés, forment un noyau hétérocyclique à 5 à 7 chaînons, qui peut contenir au moins un hétéroatome ou groupe contenant un hétéroatome supplémentaire, choisi parmi O, S, NR³ et C=O, R³ étant choisi parmi un hydrogène, un alkyle, un cycloalkyle et un aryle,
ou X est un groupe CR⁴R⁵ et R¹ et R⁴, conjointement avec l'atome de carbone auquel ils sont liés, forment un noyau carbocyclique à 3 à 7 chaînons,
pour améliorer la sélectivité et/ou le taux de conversion de la réaction de fluoration,
dans laquelle un complexe de HF choisi parmi les complexes de polyhydrofluorure de trialkylamines et de fluorures de tétraalkylammonium est utilisé comme agent de fluoration et dans laquelle l'électrode à couche de diffusion gazeuse est utilisée comme anode.

8. Utilisation selon la revendication 7 pour la fluoration électrochimique de carbonate d'éthylène afin d'obtenir un produit de fluoration contenant du carbonate de 4-fluoroéthylène.

9. Utilisation selon la revendication 7 pour la fluoration électrochimique de carbonate de propylène afin d'obtenir un produit de fluoration contenant du carbonate de 4-fluoropropylène.
